# EUROPÄISCHE PATENTANMELDUNG

(11) **EP 1 106 622 A2**
(43) Veröffentlichungstag der Anmeldung: **13.06.2001**
(21) Anmeldenummer: 00122746.1
(22) Anmeldetag: 19.10.2000
(51) Int. Cl.: C07H 21/00, C12P 13/04, C12P 13/08

(54) **Neue für das pfkA-Gen codierende Nukleotidsequenzen**

(30) Priorität: 23.11.1999 DE 19956133; 11.03.2000 DE 10011922
(71) Anmelder: Degussa AG, 40474 Düsseldorf (DE)
(72) Erfinder: Möckel, Bettina, Dr., 40597 Düsseldorf (DE); Pfefferle, Walter, Dr., 33790 Halle (Westf.) (DE)

(57) **Zusammenfassung**

Die Erfindung betrifft ein isoliertes Polynukleotid, enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukleotid, das für ein Polypeptid codiert, das eine Aminosäuresequenz enthält, die zu mindestens 70 % identisch ist mit der Aminosäuresequenz von SEQ ID No.2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c),
Verfahren zur fermentativen Herstellung von L-Aminosäuren unter Verstärkung des pfkA-Gens und die Verwendung als Primer oder Hybridisierungssonde.

## Beschreibung

Gegenstand der Erfindung sind für das pfkA-Gen kodierende Nukleotidsequenzen und Verfahren zur fermentativen Herstellung von Aminosäuren, insbesondere L-Lysin, unter Verwendung von coryneformen Bakterien, in denen das pfkA-Gen verstärkt wird.

### Stand der Technik

Aminosäuren, insbesondere L-Lysin, finden in der Humanmedizin und in der pharmazeutischen Industrie, insbesondere aber in der Tierernährung, Anwendung.

Es ist bekannt, daß Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensbesserungen können fermentationstechnische Maßnahmen wie z. B. Rührung und Versorgung mit Sauerstoff, oder die Zusammensetzung der Nährmedien wie z. B. die Zuckerkonzentration während der Fermentation, oder die Aufarbeitung zur Produktform durch z. B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z. B. das Lysin-Analogon S-(2-Aminoethyl)-Cystein oder auxotroph für regulatorisch bedeutsame Metabolite sind und L-Aminosäuren wie z. B. L-Lysin produzieren.

Seit einigen Jahren werden ebenfalls Methoden der rekombinanten DNA-Technik zur Stammverbesserung Aminosäure produzierender Stämme von Corynebacterium eingesetzt, indem man einzelne Aminosäure-Biosynthesegene amplifiziert und die Auswirkung auf die Aminosäure-Produktion untersucht. Übersichtsartikel hierzu findet man unter anderem bei Kinoshita ("Glutamic Acid Bacteria", in: Biology of Industrial Microorganisms, Demain and Solomon (Eds.), Benjamin Cummings, London, UK, 1985, 115-142), Hilliger (BioTec 2, 40-44 (1991)), Eggeling (Amino Acids 6:261-272 (1994)), Jetten und Sinskey (Critical Reviews in Biotechnology 15, 73-103 (1995)) und Sahm et al. (Annuals of the New York Academy of Science 782, 25-39 (1996)).

### Aufgabe der Erfindung

Die Erfinder haben sich zur Aufgabe gestellt, neue Maßnahmen zur verbesserten fermentativen Herstellung von Aminosäuren, insbesondere L-Lysin, bereitzustellen.

### Beschreibung der Erfindung

Aminosäuren, insbesondere L-Lysin, finden in der Humanmedizin, in der pharmazeutischen Industrie und insbesondere in der Tierernährung Anwendung. Es besteht daher ein allgemeines Interesse daran, neue verbesserte Verfahren zur Herstellung von Aminosäuren, insbesondere L-Lysin, bereitzustellen.

Wenn im folgenden L-Lysin oder Lysin erwähnt werden, sind damit nicht nur die Base, sondern auch die Salze wie z. B. Lysin-Monohydrochlorid oder Lysin-Sulfat gemeint.

Gegenstand der Erfindung ist ein isoliertes Polynukleotid aus coryneformen Bakterien, enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukleotid, das für ein Polypeptid codiert, das eine Aminosäuresequenz enthält, die zu mindestens 70% identisch ist mit der Aminosäuresequenz von SEQ ID No. 2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c).

Gegenstand der Erfindung ist ebenfalls das Polynukleotid gemäß Anspruch 1, wobei es sich bevorzugt um eine replizierbare DNA handelt, enthaltend:
(i) die Nukleotidsequenz, gezeigt in SEQ ID No. 1, oder
(ii) mindestens eine Sequenz, die der Sequenz (i) innerhalb des Bereichs der Degeneration des genetischen Codes entspricht, oder
(iii) mindestens eine Sequenz, die mit der zur Sequenz (i) oder (ii) komplementären Sequenz hybridisiert, und gegebenenfalls
(iv) funktionsneutralen Sinnmutationen in (i).

Weitere Gegenstände sind
ein Polynukleotid gemäß Anspruch 4, enthaltend die Nukleotidsequenz wie in SEQ ID No. 1 dargestellt,
ein Polynukleotid gemäß Anspruch 6, das für ein Polypeptid codiert, das die Aminosäuresequenz, wie in SEQ ID No. 2 dargestellt, enthält
ein Vektor, enthaltend das Polynukleotid gemäß Anspruch 1, insbesondere Pendelvektor oder Plasmidvektor
und als Wirtszelle dienende coryneforme Bakterien, die den Vektor enthalten.

Gegenstand der Erfindung sind ebenso Polynukleotide, die im wesentlichen aus einer Polynukleotidsequenz bestehen, die erhältlich sind durch Screening mittels Hybridisierung einer entsprechenden Genbank, die das vollständige Gen mit der Polynukleotidsequenz entsprechend SEQ ID No. 1 enthalten, mit einer Sonde, die die Sequenz des genannten Polynukleotids gemäß SEQ ID No. 1 oder ein Fragment davon enthält und Isolierung der genannten DNA-Sequenz.

Polynukleotidsequenzen gemäß der Erfindung sind als Hybridisierungs-Sonden für RNA, cDNA und DNA geeignet, um cDNA in voller Länge zu isolieren, die für Phosphofructokinase codieren und solche cDNA oder Gene zu isolieren, die eine hohe Ähnlichkeit der Sequenz mit der des Phosphofructokinase-Gens aufweisen.

Polynukleotidsequenzen gemäß der Erfindung sind weiterhin geeignet als Primer zur Herstellung von DNA von Genen, die für Phosphofructokinase codieren, durch die Polymerase-Kettenreaktion (PCR).

Solche als Sonden oder Primer dienende Oligonukleotide enthalten mindestens 30, bevorzugt mindestens 20, ganz besonders bevorzugt mindestens 15 aufeinanderfolgende Nukleotide. Geeignet sind ebenfalls Oligonukleotide mit einer Länge von mindestens 40 oder 50 Nukleotiden.

"Isoliert" bedeutet aus seinem natürlichen Umfeld herausgetrennt.

"Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Unter "Polypeptiden" versteht man Peptide oder Proteine, die zwei oder mehr über Peptidbindungen verbundene Aminosäuren enthalten.

Die Polypeptide gemäß Erfindung schließen ein Polypeptid gemäß SEQ ID No. 2, insbesondere solche mit der biologischen Aktivität der Phosphofructokinase und auch solche ein, die zu wenigstens 70 % identisch sind mit dem Polypeptid gemäß SEQ ID No. 2, bevorzugt zu wenigstens 80% und besonders die zu wenigstens 90 % bis 95 % Identität mit dem Polypeptid gemäß SEQ ID No. 2 und die genannte Aktivität aufweisen.

Die Erfindung betrifft weiterhin ein Verfahren zur fermentativen Herstellung von Aminosäuren, insbesondere L-Lysin, unter Verwendung von coryneformen Bakterien, die insbesondere bereits eine Aminosäure produzieren, und in denen die für das pfkA-Gen codierenden Nukleotidsequenzen verstärkt, insbesondere überexprimiert werden.

Der Begriff "Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man beispielsweise die Kopienzahl des Gens bzw. der Gene erhöht, einen starken Promotor verwendet oder ein Gen verwendet, das für ein entsprechendes Enzym mit einer hohen Aktivität kodiert und gegebenenfalls diese Maßnahmen kombiniert.

Die Mikroorganismen, die Gegenstand der vorliegenden Erfindung sind, können L-Aminosäuren, insbesondere L-Lysin, aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind die zum Beispiel bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Corynebacterium melassecola ATCC17965
Brevibacterium flavum ATCC14067
Brevibacterium lactofermentum ATCC13869 und
Brevibacterium divaricatum ATCC14020
und daraus hergestellte L-Lysin produzierende Mutanten bzw. Stämme, wie beispielsweise
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 und
Corynebacterium glutamicum DSM5715.

Den Erfindern gelang es, das neue, für das Enzym Phosphofructokinase (EC 2.7.1.11) kodierende pfkA-Gen von C. glutamicum zu isolieren.

Zur Isolierung des pfkA-Gens oder auch anderer Gene von C. glutamicum wird zunächst eine Genbank dieses Mikroorganismus in E. coli angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495-508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E. coli K-12 Stamm NM554 (Raleigh et al., 1988, Nucleic Acids Research 16:1563-1575) angelegt wurde. Börmann et al. (Molecular Microbiology 6(3), 317-326 (1992)) wiederum beschreiben eine Genbank von C. glutamicum ATCC13032 unter Verwendung des Cosmids pHC79 (Hohn und Collins, Gene 11, 291-298 (1980)). Zur Herstellung einer Genbank von C. glutamicum in E. coli können auch Plasmide wie pBR322 (Bolivar, Life Sciences, 25, 807-818 (1979)) oder pUC9 (Vieira et al., 1982, Gene, 19:259-268) verwendet werden. Als Wirte eignen sich besonders solche E. coli-Stämme, die restriktions- und rekombinationsdefekt sind. Ein Beispiel hierfür ist der Stamm DH5amcr, der von Grant et al. (Proceedings of the National Academy of Sciences USA, 87 (1990) 4645-4649) beschrieben wurde. Die mit Hilfe von Cosmiden klonierten langen DNA-Fragmente können anschließend wiederum in gängige, für die Sequenzierung geeignete Vektoren subkloniert und anschließend sequenziert werden, so wie es z. B. bei Sanger et al. (Proceedings of the National Academy of Sciences of the United States of America, 74:5463-5467, 1977) beschrieben ist.

Auf diese Weise wurde die neue für das Gen pfkA kodierende DNA-Sequenz von C. glutamicum erhalten, die als SEQ ID NO 1 Bestandteil der vorliegenden Erfindung ist. Weiterhin wurde aus der vorliegenden DNA-Sequenz mit den oben beschriebenen Methoden die Aminosäuresequenz des entsprechenden Proteins abgeleitet. In SEQ ID NO 2 ist die sich ergebende Aminosäuresequenz des pfkA-Genproduktes dargestellt.

Kodierende DNA-Sequenzen, die sich aus SEQ ID NO 1 durch die Degeneriertheit des genetischen Codes ergeben, sind ebenfalls Bestandteil der Erfindung. In gleicher Weise sind DNA-Sequenzen, die mit SEQ ID NO 1 oder Teilen von SEQ ID NO 1 hybridisieren, Bestandteil der Erfindung. In der Fachwelt sind weiterhin konservative Aminosäureaustausche wie z. B. Austausch von Glycin gegen Alanin oder von Asparaginsäure gegen Glutaminsäure in Proteinen als "Sinnmutationen" (sense mutations) bekannt, die zu keiner grundsätzlichen Veränderung der Aktivität des Proteins führen, d. h. funktionsneutral sind. Weiterhin ist bekannt, daß Änderungen am N- und/oder C-Terminus eines Proteins dessen Funktion nicht wesentlich beeinträchtigen oder sogar stabilisieren können. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie. Aminosäuresequenzen, die sich in entsprechender Weise aus SEQ ID NO 2 ergeben, sind ebenfalls Bestandteil der Erfindung.

In gleicher Weise sind DNA-Sequenzen, die mit SEQ ID NO 1 oder Teilen von SEQ ID NO 1 hybridisieren Bestandteil der Erfindung. Schließlich sind DNA-Sequenzen Bestandteil der Erfindung, die durch die Polymerase-Kettenreaktion (PCR) unter Verwendung von Primern hergestellt werden, die sich aus SEQ ID NO 1 ergeben. Derartige Oligonukleotide haben typischerweise eine Länge von mindestens 15 Nukleotiden.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260). Anleitungen zur Amplifikation von DNA-Sequenzen mit Hilfe der Polymerase-Kettenreaktion (PCR) findet der Fachmann unter anderem im Handbuch von Gait: Oligonukleotide synthesis: a practical approach (IRL Press, Oxford, UK, 1984) und bei Newton und Graham: PCR (Spektrum Akademischer Verlag, Heidelberg, Deutschland, 1994).

Die Erfinder fanden heraus, daß coryneforme Bakterien nach Überexpression des pfkA-Gens in verbesserter Weise Aminosäuren, insbesondere L-Lysin, produzieren.

Zur Erzielung einer Überexpression kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Lysin-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EPS 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15 - 24 (1993)), in der japanischen Offenlegungsschrift JP-A-10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

Beispielhaft wurde das erfindungsgemäße pfkA-Gen mit Hilfe von Plasmiden überexprimiert.

Als Plasmide eignen sich solche, die in coryneformen Bakterien repliziert werden. Zahlreiche bekannte Plasmidvektoren wie z. B. pZ1 (Menkel et al., Applied and Environmental Microbiology (1989) 64: 549-554), pEKEx1 (Eikmanns et al., Gene 102:93-98 (1991)) oder pHS2-1 (Sonnen et al., Gene 107:69-74 (1991)) beruhen auf den kryptischen Plasmiden pHM1519, pBL1 oder pGA1. Andere Plasmidvektoren wie z. B. solche, die auf pCG4 (US-A 4,489,160), oder pNG2 (Serwold-Davis et al., FEMS Microbiology Letters 66, 119-124 (1990)), oder pAG1 (US-A 5,158,891) beruhen, können in gleicher Weise verwendet werden.

Weiterhin eignen sich solche Plasmidvektoren mit Hilfe derer man das Verfahren der Genamplifikation durch Integration in das Chromosom anwenden kann, so wie es beispielsweise von Reinscheid et al. (Applied and Environmental Microbiology 60, 126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise E. coli), nicht aber in C. glutamicum replizieren kann. Als Vektoren kommen bespielsweise pSUP301 (Simon et al., Bio/Technology 1, 784-791 (1983)), pK18mob oder pK19mob (Schäfer et al., Gene 145, 69-73 (1994)), pGEM-T (Promega corporation, Madison, WI, USA), pCR2.1-TOPO (Shuman (1994). Journal of Biological Chemistry 269:32678-84; US-A 5,487,993), pCR®Blunt (Firma Invitrogen, Groningen, Niederlande; Bernard et al., Journal of Molecular Biology, 234: 534-541 (1993)) oder pEM1 (Schrumpf et al, 1991, Journal of Bacteriology 173:4510-4516) in Frage. Der Plasmidvektor, der das zu amplifizierende Gen enthält, wird anschließend durch Konjugation oder Transformation in den gewünschten Stamm von C. glutamicum überführt. Die Methode der Konjugation ist beispielsweise bei Schäfer et al. (Applied and Environmental Microbiology 60, 756-759 (1994)) beschrieben. Methoden zur Transformation sind beispielsweise bei Thierbach et al. (Applied Microbiology and Biotechnology 29, 356-362 (1988)), Dunican und Shivnan (Bio/Technology 7, 1067-1070 (1989)) und Tauch et al. (FEMS Microbiological Letters 123, 343-347 (1994)) beschrieben. Nach homologer Rekombination mittels eines "cross over"-Ereignisses enthält der resultierende Stamm mindestens zwei Kopien des betreffenden Gens.

Zusätzlich kann es für die Produktion von Aminosäuren, insbesondere L-Lysin vorteilhaft sein, neben dem pfkA-Gen eines oder mehrere Enzyme des jeweiligen Biosyntheseweges, der Glykolyse, der Anaplerotik, des Zitronensäure-Zyklus oder des Aminosäure-Exports zu verstärken oder zu überexprimieren.

So kann beispielsweise für die Herstellung von L-Lysin gleichzeitig eines oder mehrere der Gene, ausgewählt aus der Gruppe
- das für die Dihydrodipicolinat-Synthase kodierende dapA-Gen (EP-B 0 197 335), oder
- das für die Glyceraldehyd-3-Phosphat Dehydrogenase kodierende gap-Gen (Eikmanns (1992), Journal of Bacteriology 174:6076-6086), oder
- das für die Triosephosphat Isomerase codierende tpi-Gen (Eikmanns (1992), Journal of Bacteriology 174:6076-6086), oder
- das für die 3-Phosphoglycerat Kinase codierende pgk-Gen (Eikmanns (1992), Journal of Bacteriology 174:6076-6086), oder
- das für die Pyruvat Carboxylase codierende pyc-Gen (Eikmanns (1992), Journal of Bacteriology 174:6076-6086), oder
- das für den Lysin-Export kodierende lysE-Gen (DE-A-195 48 222)
überexprimiert werden.

Weiterhin kann es für die Produktion von Aminosäuren, insbesondere L-Lysin, vorteilhaft sein, neben der Verstärkung des pfkA-Gens gleichzeitig
- das für die Phosphoenolpyruvat-Carboxykinase codierende pck-Gen (DE 199 50 409.1 DSM 13047) und/oder
- das für die Glucose-6-Phosphat Isomerase kodierende pgi-Gen (US 09/396,478, DSM 12969)
abzuschwächen.

Weiterhin kann es für die Produktion von Aminosäuren, insbesondere L-Lysin, vorteilhaft sein, neben der Überexpression des pfkA-Gens unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Die erfindungsgemäß hergestellten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von Aminosäuren, insbesondere L-Lysin kultiviert werden. Eine Zusammenfassung über bekannte Kultivierungsmethoden sind im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z. B. Glucose, Saccharose, Lactose, Fructose, Maltose, Melasse, Stärke und Cellulose, Öle und Fette wie z. B. Sojaöl, Sonnenblumenöl, Erdnußöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muß weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten, werden Sauerstoff oder Sauerstoffhaltige Gasmischungen wie z. B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum an Lysin gebildet hat. Dieses Ziel wird normalerweise innerhalb von 10 Stunden bis 160 Stunden erreicht.

Die Analyse von L-Lysin kann durch
Anionenaustauschchromatographie mit anschließender Ninhydrin Derivatisierung erfolgen, so wie bei Spackman et al. (Analytical Chemistry, 30, (1958), 1190) beschrieben.

Das erfindungsgemäße Verfahren dient zur fermentativen Herstellung von Aminosäuren, insbesondere L-Lysin.

### Beispiele

Die vorliegende Erfindung wird im folgenden anhand von Ausführungsbeispielen näher erläutert.

### Beispiel 1

### Herstellung einer genomischen Cosmid-Genbank aus Corynebacterium glutamicum ATCC 13032

Chromosomale DNA aus Corynebacterium glutamicum ATCC 13032 wurde wie bei Tauch et al. (1995, Plasmid 33:168-179) beschrieben isoliert und mit dem Restriktionsenzym Sau3AI (Amersham Pharmacia, Freiburg, Deutschland, Produktbeschreibung Sau3AI, Code no. 27-0913-02) partiell gespalten. Die DNA-Fragmente wurden mit shrimp alkalischer Phosphatase (Roche Molecular Biochemicals, Mannheim, Deutschland, Produktbeschreibung SAP, Code no. 1758250) dephosphoryliert. Die DNA des Cosmid-Vektors SuperCos1 (Wahl et al. (1987) Proceedings of the National Academy of Sciences USA 84:2160-2164), bezogen von der Firma Stratagene (La Jolla, USA, Produktbeschreibung SuperCosl Cosmid Vektor Kit, Code no. 251301) wurde mit dem Restriktionsenzym XbaI (Amersham Pharmacia, Freiburg, Deutschland, Produktbeschreibung XbaI, Code no. 27-0948-02) gespalten und ebenfalls mit shrimp alkalischer Phosphatase dephosphoryliert. Anschließend wurde die Cosmid-DNA mit dem Restriktionsenzym BamHI (Amersham Pharmacia, Freiburg, Deutschland, Produktbeschreibung BamHI, Code no. 27-0868-04) gespalten. Die auf diese Weise behandelte Cosmid-DNA wurde mit der behandelten ATCC 13032-DNA gemischt und der Ansatz mit T4-DNA-Ligase (Amersham Pharmacia, Freiburg, Deutschland, Produktbeschreibung T4-DNA-Ligase, Code no.27-0870-04) behandelt. Das Ligationsgemisch wurde anschließend mit Hilfe des Gigapack II XL Packing Extracts (Stratagene, La Jolla, USA, Produktbeschreibung Gigapack II XL Packing Extract, Code no. 200217) in Phagen verpackt. Zur Infektion des E. coli Stammes NM554 (Raleigh et al. 1988, Nucleic Acid Research 16:1563-1575) wurden die Zellen in 10 mM MgSO₄ aufgenommen und mit einem Aliquot der Phagensuspension vermischt. Infektion und Titerung der Cosmidbank wurden wie bei Sambrook et al. (1989, Molecular Cloning: A Laboratory Manual, Cold Spring Harbor) beschrieben durchgeführt, wobei die Zellen auf LB-Agar (Lennox, 1955, Virology, 1:190) mit 100 ug/ml Ampicillin ausplattiert wurden. Nach Inkubation über Nacht bei 37°C wurden rekombinante Einzelklone selektioniert.

### Beispiel 2

### Isolierung und Sequenzierung des pfkA-Gens

Die Cosmid-DNA einer Einzelkolonie wurde mit dem Qiaprep Spin Miniprep Kit (Product No. 27106, Qiagen, Hilden, Germany) nach Herstellerangaben isoliert und mit dem Restriktionsenzym Sau3AI (Amersham Pharmacia, Freiburg, Deutschland, Produktbeschreibung Sau3AI, Product No. 27-0913-02) partiell gespalten. Die DNA-Fragmente wurden mit shrimp alkalischer Phosphatase (Roche Molecular Biochemicals, Mannheim, Deutschland, Produktbeschreibung SAP, Product No. 1758250) dephosphoryliert. Nach gelelektrophoretischer Auftrennung erfolgte die Isolierung der Cosmidfragmente im Größenbereich von 1500 bis 2000 bp mit dem QiaExII Gel Extraction Kit (Product No. 20021, Qiagen, Hilden, Germany). Die DNA des Sequenziervektors pZero-1 bezogen von der Firma Invitrogen (Groningen, Niederlande, Produktbeschreibung Zero Background Cloning Kit, Product No. K2500-01) wurde mit dem Restriktionsenzym BamHI (Amersham Pharmacia, Freiburg, Deutschland, Produktbeschreibung BamHI, Product No. 27-0868-04) gespalten. Die Ligation der Cosmidfragmente in den Sequenziervektor pZero-1 wurde wie von Sambrook et al. (1989, Molecular Cloning: A laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DHSαMCR (Grant, 1990, Proceedings of the National Academy of Sciences U.S.A., 87:4645-4649) elektroporiert (Tauch et al. 1994, FEMS Microbiol Letters, 123:343-7) und auf LB-Agar (Lennox, 1955, Virology, 1:190) mit 50 µg/ml Zeocin ausplattiert. Die Plasmidpräparation der rekombinanten Klone erfolgte mit dem Biorobot 9600 (Product No. 900200, Qiagen, Hilden, Deutschland). Die Sequenzierung erfolgte nach der Dideoxy-Kettenabbruch-Methode von Sanger et al. (1977, Proceedings of the National Academy of Sciences U.S.A., 74:5463-5467) mit Modifikationen nach Zimmermann et al. (1990, Nucleic Acids Research, 18:1067). Es wurde der "RR dRhodamin Terminator Cycle Sequencing Kit" von PE Applied Biosystems (Product No. 403044, Weiterstadt, Deutschland) verwendet. Die gelelektrophoretische Auftrennung und Analyse der Sequenzierreaktion erfolgte in einem "Rotiphorese NF Acrylamid/Bisacrylamid" Gel (29:1) (Product No. A124.1, Roth, Karlsruhe, Germany) mit dem "ABI Prism 377" Sequenziergerät von PE Applied Biosystems (Weiterstadt, Deutschland).

Die erhaltenen Roh-Sequenzdaten wurden anschließend unter Anwendung des Staden-Programpakets (1986, Nucleic Acids Research, 14:217-231) Version 97-0 prozessiert. Die Einzelsequenzen der pZero1-Derivate wurden zu einem zusammenhängenden Contig assembliert. Die computergestützte Kodierbereichsanalyse wurde mit dem Programm XNIP (Staden, 1986, Nucleic Acids Research, 14:217-231) angefertigt. Weitere Analysen wurden mit den "BLAST search programs" (Altschul et al., 1997, Nucleic Acids Research, 25:3389-3402), gegen die non-redundant Datenbank des "National Center for Biotechnology Information" (NCBI, Bethesda, MD, USA) durchgeführt.

Die erhaltene Nukleotidsequenz ist in SEQ ID NO 1 dargestellt. Die Analyse der Nukleotidsequenz ergab ein offenes Leseraster von 1029 Basenpaaren, welches als pfkA-Gen bezeichnet wurde. Das pfkA-Gen kodiert für ein Protein von 343 Aminosäuren.

### Beispiel 3

### Herstellung eines Plasmides zur Expression von pfkA in Corynebacterium glutamicum

### 3.1. Klonierung von pfkA im Vektor pCR2-Blunt

Chromosomale DNA aus Corynebacterium glutamicum ATCC 13032 wurde isoliert wie bei Tauch et al. (1995, Plasmid 33:168-179). Aufgrund der aus Beispiel 2 für C. glutamicum bekannten Sequenz des pfkA-Gens wurden die folgenden Oligonukleotide für die Polymerase Kettenreaktion ausgewählt:

pfkA-exp
5`-AAC TGC AGC TCT GGC GAT TA-3`

pfk-ex2
5`-AAC TAT CCA AAC ATT GCC TG-3`

Die dargestellten Primer wurden von der Firma MWG Biotech (Ebersberg, Deutschland) synthetisiert und nach der Standard-PCR-Methode von Innis et al. (PCR protocols. A guide to methods and applications, 1990, Academic Press) mit Pwo-Polymerase der Firma Roche Diagnostics GmbH (Mannheim, Deutschland) die PCR Reaktion durchgeführt. Mit Hilfe der Polymerase-Kettenreaktion wurde ein ca. 1160 bp großes DNA-Fragment isoliert, welches das pfkA-Gen trägt.

Das amplifizierte DNA Fragment wurde mit dem Zero Blunt PCR Cloning Kit der Firma Invitrogen Corporation (Carlsbad, CA, USA; Katalog Nummer K2700-20) in den Vektor pCR2-Blunt Vektor (Bernard et al., (1983) Journal of Molecular Biology. 234:534-541) ligiert. Anschließend wurde der E. coli Stamm ToplOF (Grand et al. (1990) Proceedings of the National Academy of Sciences, USA. 87:4645-4649) mit dem Ligationsansatz transformiert. Die Selektion von Plasmid-tragenden Zellen erfolgte durch Ausplattieren des Transformationsansatzes auf LB Agar (Sambrook et al., Molecular cloning: a laboratory manual. 2^{nd} Ed. Cold Spring Harbor Laboratory Press, Cold Spring Harbor, N.Y.), der mit 50 mg/l Kanamycin supplementiert worden war. Plasmid-DNA wurde aus einer Transformante mit Hilfe des QIAprep Spin Miniprep Kit der Firma Qiagen isoliert und durch Restriktion mit dem Restriktionsenzym EcoRI und anschließender Agarosegel-Elektrophorese (0,8%) überprüft. Das Plasmid wurde pCRB1-pfkAexp1 genannt.

### 3.2. Herstellung des Shuttle Vektors pEC-T18mob2

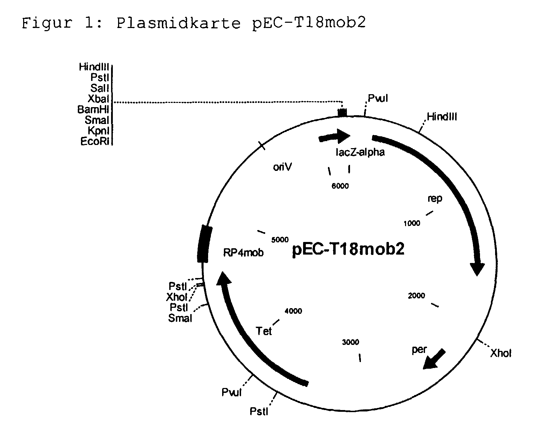

Nach dem Stand der Technik wurde der E. coli - C. glutamicum Shuttle-Vektor konstruiert. Der Vektor enthält die Replikationsregion rep des Plasmids pGA1 einschließlich des Replikationseffectors per (US-A- 5,175,108; Nesvera et al., Journal of Bacteriology 179, 1525-1532 (1997)), das Tetracyclinresistenz vermittelnde tetA(Z)-Gen des Plasmids pAGl (US-A- 5,158,891; Genbank-Eintrag beim National Center for Biotechnology Information (NCBI, Bethesda, MD, USA) mit der accession number AF121000), die Replikationsregion oriV des Plasmids pMB1 (Sutcliffe, Cold Spring Harbor Symposium on Quantitative Biology 43, 77-90 (1979)), das lacZα Genfragment einschließlich des lac-Promotors und einer Mehrfachklonierschnittstelle (multiple cloning site, mcs) (Norrander et al. Gene 26, 101-106 (1983)) und die mob-Region des Plasmids RP4 (Simon et al.,(1983) Bio/Technology 1:784-791). Der konstruierte Vektor wurde in den E. coli Stamm DH5α (Hanahan, In: DNA cloning. A practical approach. Vol. I. IRL-Press, Oxford, Washington DC, USA) transformiert. Die Selektion von Plasmid-tragenden Zellen erfolgte durch Ausplattieren des Transformationsansatzes auf LB Agar (Sambrook et al., Molecular cloning: a laboratory manual. 2^{nd} Ed. Cold Spring Harbor Laboratory Press, Cold Spring Harbor, N.Y.), der mit 5 mg/l Tetracyclin supplementiert worden war. Plasmid-DNA wurde aus einer Transformante mit Hilfe des QIAprep Spin Miniprep Kit der Firma Qiagen isoliert und durch Restriktion mit dem Restriktionsenzym EcoRI und HindIII anschließender Agarosegel-Elektrophorese (0,8%) überprüft. Das Plasmid wurde pEC-T18mob2 genannt und ist in Figur 1 dargestellt.

### 3.3. Klonierung von pfkA im Shuttle-Vektor pEC-T18mob2

Als Vektor wurde der in Beispiel 3.2 beschriebene E. coli-C. glutamicum Shuttle-Vektor pEC-T18mob2 verwendet. DNA dieses Plasmids wurde mit dem Restriktionsenzym EcoRI vollständig gespalten und anschließend mit shrimp alkalischer Phosphatase (Roche Molecular Biochemicals, Mannheim, Deutschland, Produktbeschreibung SAP, Product No. 1758250) dephosphoryliert.

Aus dem in Beispiel 3.1. beschriebenen Plasmid pCRB1-pfkAexp1 wurde das pfkA-Gen durch vollständige Spaltung mit dem Enzym EcoRI isoliert. Das ca 1160bp große pfkA Fragment wurde aus dem Agarosegel mit dem QiaExII Gel Extraction Kit (Product No. 20021, Qiagen, Hilden, Germany) isoliert.

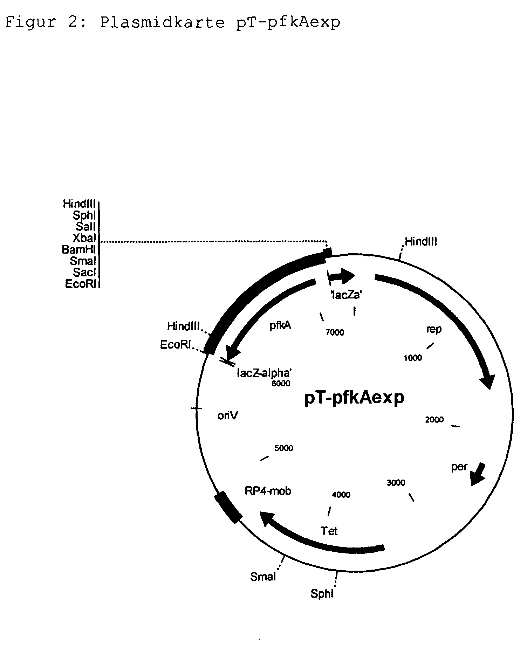

Das auf diese Weise gewonnene pfkA-Fragment wurde mit dem vorbereiteten Vektor pEC-T18mob2 gemischt und der Ansatz mit T4-DNA-Ligase (Amersham Pharmacia, Freiburg, Deutschland, Produktbeschreibung T4-DNA-Ligase, Code no.27-0870-04) behandelt. Der Ligationsansatz wurde in den E. coli Stamm DH5α(Hanahan, In: DNA cloning. A practical approach. Vol. I. IRL-Press, Oxford, Washington DC, USA) transformiert. Die Selektion von Plasmid-tragenden Zellen erfolgte durch Ausplattieren des Transformationsansatzes auf LB-Agar (Lennox, 1955, Virology, 1:190) mit 5 mg/l Tetracyclin. Nach Inkubation über Nacht bei 37°C wurden rekombinante Einzelklone selektioniert. Plasmid DNA wurde aus einer Transformante mit dem Qiaprep Spin Miniprep Kit (Product No. 27106, Qiagen, Hilden, Germany) nach Herstellerangaben isoliert und mit dem Restriktionsenzym EcoRI gespalten, um das Plasmid durch anschließende Agarosegel-Elektrophorese zu überprüfen. Das erhaltene Plasmid wurde pT-pfkAexp genannt. Es ist in Figur 2 dargestellt.

### Beispiel 4:

### Transformation des Stammes DSM5715 mit dem Plasmid pT-pfkAexp

Der Stamm DSM5715 (EP-B- 0435 132) wurde mit dem Plasmid pT-pfkAexp unter Anwendung der von Liebl et al., (FEMS Microbiology Letters, 53:299-303 (1989)) beschriebenen Elektroporationsmethode transformiert. Die Selektion der Transformanten erfolgte auf LBHIS Agar bestehend aus 18,5 g/l Brain-Heart Infusion Boullion, 0,5M Sorbitol, 5 g/l Bacto-Trypton, 2,5 g/l Bacto-yeast-extract, 5 g/l NaCl und 18 g/l Bacto-Agar, der mit 5 mg/l Tetracyclin supplementiert worden war. Die Inkubation erfolgte für 2 Tage bei 33°C.

Plasmid DNA wurde aus einer Transformante nach den üblichen Methoden isoliert (Peters-Wendisch et al., 1998, Microbiology, 144, 915 - 927), mit der Restriktionsendonuklease EcoRI geschnitten um das Plasmid durch anschließende Agarosegel-Elektrophorese zu überprüfen. Der erhaltene Stamm wurde DSM5715/pT-pfkAexp genannt.

### Beispiel 5

### Herstellung von Lysin

Der in Beispiel 4 erhaltene C. glutamicum Stamm
DSM5715/pT-pfkAexp wurde in einem zur Produktion von Lysin geeigneten Nährmedium kultiviert und der Lysingehalt im Kulturüberstand bestimmt.

Dazu wurde der Stamm zunächst auf Agarplatte mit dem entsprechenden Antibiotikum (Hirn-Herz Agar mit Tetracyclin (5 mg/l) für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium CgIII (2,5 g/l NaCl, 10 g/l Bacto-Pepton, 10 g/l Bacto-Yeast Extract, 20 g/l Glucose, pH 7,4)verwendet. Diesem wurde Tetracyclin (5 mg/l) zugesetzt. Die Vorkultur wurde 16 Stunden bei 33°C bei 240 rpm auf dem Schüttler inkubiert. Von dieser Vorkultur wurde eine Hauptkultur angeimpft, so daß die Anfangs-OD (660nm) der Hauptkultur 0,1 betrug. Für die Hauptkultur wurde das Medium MM verwendet.

| Medium MM | |
|---|---|
| CSL (Corn Steep Liquor) | 5 g/l |
| MOPS (Morpholino-Propansulfonsäure) | 20 g/l |
| Glucose(getrennt autoklaviert) | 50 g/l |
| (NH₄)₂SO₄ | 25 g/l |
| KH₂PO₄ | 0,1 g/l |
| MgSO4 * 7 H₂O | 1,0 g/l |
| CaCl₂ * 2 H₂O | 10 mg/l |
| FeSO₄ * 7 H₂O | 10 mg/l |
| MnSO₄ * H₂O | 5,0 mg/1 |
| Biotin (sterilfiltriert) | 0,3 mg/l |
| Thiamin * HCl (sterilfiltriert) | 0,2 mg/l |
| L-Leucin (sterilfiltriert) | 0,1 g/l |
| CaCO₃ | 25 g/l |

CSL, MOPS und die Salzlösung wurden mit Ammoniakwasser auf pH 7 eingestellt und autoklaviert. Anschließend wurden die sterilen Substrat- und Vitaminlösungen zugesetzt, sowie das trocken autoklavierte CaCO₃.

Die Kultivierung erfolgt in 10 ml Volumen in einem 100 ml Erlenmeyerkolben mit Schikanen. Es wurde Kanamycin (25 mg/l) zugesetzt. Die Kultivierung erfolgte bei 33°C und 80% Luftfeuchte.

Nach 24 Stunden wurde die OD bei einer Meßwellenlänge von 660 nm mit dem Biomek 1000 (Beckmann Instruments GmbH, München) ermittelt. Die gebildete Lysinmenge wurde mit einem Aminosäureanalysator der Firma Eppendorf-BioTronik (Hamburg, Deutschland) durch Ionenaustauschchromatographi und Nachsäulenderivatisierung mit Ninhydrindetektion bestimmt.

In Tabelle 1 ist das Ergebnis des Versuchs dargestellt.

**Tabelle 1**

| Stamm | OD(660) | Lysin-HCl g/l |
|---|---|---|
| DSM5715/pT-pfkAexp | 14,6 | 10,1 |
| DSM5715 | 15,2 | 8,1 |

Folgende Figuren sind beigefügt:
Figur 1: Karte des Plasmids pT-pfkAexp
Figur 2: Karte des Plasmids pT-pfkAexp

Die verwendeten Abkürzungen und Bezeichnungen haben folgende Bedeutung.

| | |
|---|---|
| Tet: | Resistenzgen für Tetracyclin |
| oriV: | Plasmidkodierter Replikationsursprung von E. |
| | coli |
| RP4mob: | mob Region zur Mobilisierung des Plasmides |
| rep: | Plasmidkodierter Replikationsursprung aus |
| | C. glutamicum Plasmid pGA1 |
| per: | Gen zur Kontrolle der Kopienzahl aus pGA1 |
| lacZ-alpha: | lacZα-Genfragment (N-Terminus) des |
| | β-Galactosidase Gens |
| 'lacZa': | 5'Ende des lacZα-Genfragments |
| lacZ-alpha': | 3'Ende des lacZα-Genfragments |
| pfkA: | pfkA-Gen aus C. glutamicum ATCC13032 |
| BamHI: | Schnittstelle des Restriktionsenzyms BamHI |
| EcoRI: | Schnittstelle des Restriktionsenzyms EcoRI |
| HindIII: | Schnittstelle des Restriktionsenzyms HindIII |
| KpnI: | Schnittstelle des Restriktionsenzyms KpnI |
| PstI: | Schnittstelle des Restriktionsenzyms PstI |
| PvuI: | Schnittstelle des Restriktionsenzyms PvuI |
| SalI: | Schnittstelle des Restriktionsenzyms SalI |
| SacI: | Schnittstelle des Restriktionsenzyms SacI |
| SmaI: | Schnittstelle des Restriktionsenzyms SmaI |
| SphI: | Schnittstelle des Restriktionsenzyms SphI |
| XbaI: | Schnittstelle des Restriktionsenzyms XbaI |
| XhoI: | Schnittstelle des Restriktionsenzyms XhoI |

## Patentansprüche

1. Isoliertes Polynukleotid aus coryneformen Bakterien, enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukleotid, das für ein Polypeptid codiert, das eine Aminosäuresequenz enthält, die zu mindestens 70 % identisch ist mit der Aminosäuresequenz von SEQ ID No.2,
c) Polynukleotid, das komplementär ist zu den Polynukleotiden von a) oder b), und
e) Polynukleotid, enthaltend mindestens 15 aufeinanderfolgende Basen der Polynukleotidsequenz von a), b) oder c).

2. Polynukleotid gemäß Anspruch 1,
wobei das Polynukleotid eine in coryneformen Bakterien replizierbare, bevorzugt rekombinante DNA ist.

3. Polynukleotid gemäß Anspruch 1,
wobei das Polynukleotid eine RNA ist.

4. Polynukleotid gemäß Anspruch 2,
enthaltend die Nukleinsäuresequenz wie in SEQ ID No. 1 dargestellt.

5. Replizierbare DNA gemäß Anspruch 2,
enthaltend
(i) die Nukleotidsequenz, gezeigt in SEQ ID No. 1, oder
(ii) mindestens eine Sequenz, die der Sequenz (i) innerhalb des Bereichs der Degeneration des genetischen Codes entspricht, oder
(iii) mindestens eine Sequenz, die mit der zur Sequenz (i) oder (ii) komplementären Sequenz hybridisiert, und gegebenenfalls
(iv) funktionsneutrale Sinnmutationen in (i).

6. Polynukleotidsequenz gemäß Anspruch 2, das für ein Polypeptid codiert, das die Aminosäuresequenz in SEQ ID No. 2 darstellt, enthält.

7. Verfahren zur fermentativen Herstellung von L-Aminosäuren, insbesondere L-Lysin,
**dadurch gekennzeichnet,**
daß man folgende Schritte durchführt:
a) Fermentation der die L-Aminosäure produzierenden coryneformen Bakterien, in denen man zumindest das pfkA-Gen oder dafür codierende Nukleotidsequenzen verstärkt, insbesondere überexprimiert.
b) Anreicherung von der L-Aminosäure im Medium oder in den Zellen der Bakterien und
c) Isolieren von der L-Aminosäure.

8. Verfahren gemäß Anspruch 7,
**dadurch gekennzeichnet,**
daß man Bakterien einsetzt, in denen man zusätzlich weitere Gene des Biosyntheseweges der gewünschten L-Aminosäure verstärkt.

9. Verfahren gemäß Anspruch 7,
**dadurch gekennzeichnet,**
daß man Bakterien einsetzt, in denen die Stoffwechselwege zumindest teilweise ausgeschaltet sind, die die Bildung des L-Lysins verringern.

10. Verfahren gemäß Anspruch 7,
**dadurch gekennzeichnet,**
daß man einen mit einem Plasmidvektor transformierten Stamm einsetzt, und der Plasmidvektor die für das pfkA-Gen codierende Nukleotidsequenz trägt.

11. Verfahren gemäß einem oder mehreren der Ansprüche 7 bis 10,
**dadurch gekennzeichnet,**
daß man coryneforme Bakterien verwendet, die L-Lysin herstellen.

12. Verfahren gemäß Anspruch 6,
**dadurch gekennzeichnet,**
daß man für die Herstellung von Lysin Bakterien fermentiert, in denen man gleichzeitig eines oder mehrere der Gene, ausgewählt aus der Gruppe
12.1 das für die Dihydrodipicolinat-Synthase kodierende dapA-Gen,
12.2 das für die Pyruvat-Carboxylase kodierende pyc-Gen,
12.3 das für die Triosephosphat Isomerase kodierende tpi-Gen,
12.4 das Gen für die Succinyldiaminopimelat-Desuccinylase kodierende dapE-Gen,
12.5 das für die Glyceraldehyd-3-Phosphat Dehydrogenase kodierende gap-Gen,
12.6 das für die 3-Phosphoglycerat Kinase kodierende pgk-Gen,
12.7 das für den Lysin-Export kodierende lysE-Gen,
gleichzeitig verstärkt, insbesondere überexprimiert oder amplifiziert.

13. Verfahren gemäß Anspruch 9,
**dadurch gekennzeichnet,**
daß man für die Herstellung von L-Lysin Bakterien fermentiert, in denen man gleichzeitig eines oder mehrere der Gene, ausgewählt aus der Gruppe
13.1 das für die Phosphoenolpyruvat-Carboxykinase codierende pck-Gen
13.2 das für die Glucose-6-Phosphat Isomerase kodierende pgi-Gen
gleichzeitig abschwächt.

14. Verfahren gemäß einem oder mehreren der vorhergehenden Ansprüche,
**dadurch gekennzeichnet,**
daß man Mikroorganismen der Gattung Corynebacterium glutamicum einsetzt.

15. Verwendung von Polynukleotidsequenzen gemäß Anspruch 1 als Primer zur Herstellung der DNA von Genen, die für die für die Phosphofructokinase codieren, durch die Polymerase-Kettenreaktion.

16. Verwendung von Polynukleotidsequenzen gemäß Anspruch 1 als Hybridisierungssonden.


SEQUENZPROTOKOLL

<110> Degussa-Hüls AG

<120> Neue für das pfkA-Gen codierende Nukleotidsequenzen

<130> 990169 BT

<140>
<141>

<160> 2

<170> PatentIn Ver. 2.1

<210> 1
<211> 1274
<212> DNA
<213> Corynebacterium glutamicum

<220>
<221> CDS
<222> (143)..(1171)

<400> 1
gtcgatttgt taatgaaact gcagctctgg cgattaaata agatggtcag agacagtttt 60

ttggcctgtc aacccctgtg attctcttat ttttgggtga ttgttccggc gcgggtgttg 120

tgatgggttt aatatggaag ac atg cga att gct act ctc acg tca ggc ggc 172
                         Met Arg Ile Ala Thr Leu Thr Ser Gly Gly
                           1               5                  10

gac tgc ccc gga cta aac gcc gtc atc cga gga atc gtc cgc aca gcc 220
Asp Cys Pro Gly Leu Asn Ala Val Ile Arg Gly Ile Val Arg Thr Ala
                 15                  20                  25

agc aat gaa ttt ggc tcc acc gtc gtt ggt tat caa gac ggt tgg gaa 268
Ser Asn Glu Phe Gly Ser Thr Val Val Gly Tyr Gln Asp Gly Trp Glu
             30                  35                  40

gga ctg tta ggc gat cgt cgc gta cag ctg tat gac gat gaa gat att 316
Gly Leu Leu Gly Asp Arg Arg Val Gln Leu Tyr Asp Asp Glu Asp Ile
         45                  50                  55

gac cga atc ctc ctt cga ggc ggc acc att ttg ggc act ggt cgc ctc 364
Asp Arg Ile Leu Leu Arg Gly Gly Thr Ile Leu Gly Thr Gly Arg Leu
     60                  65                  70

cat ccg gac aag ttt aag gcc gga att gat cag att aag gcc aac tta 412
His Pro Asp Lys Phe Lys Ala Gly Ile Asp Gln Ile Lys Ala Asn Leu
 75                  80                  85                  90

gaa gac gcc ggc atc gat gcc ctt atc cca atc ggt ggc gaa gga acc 460
Glu Asp Ala Gly Ile Asp Ala Leu Ile Pro Ile Gly Gly Glu Gly Thr
                 95                 100                 105

ctg aag ggt gcc aag tgg ctg tct gat aac ggt atc cct gtt gtc ggt    508
Leu Lys Gly Ala Lys Trp Leu Ser Asp Asn Gly Ile Pro Val Val Gly
            110                 115                 120

gtc cca aag acc att gac aat gac gtg aat ggc act gac ttc acc ttc    556
Val Pro Lys Thr Ile Asp Asn Asp Val Asn Gly Thr Asp Phe Thr Phe
        125                 130                 135

ggt ttc gat act gct gtg gca gtg gct acc gac gct gtt gac cgc ctg    604
Gly Phe Asp Thr Ala Val Ala Val Ala Thr Asp Ala Val Asp Arg Leu
    140                 145                 150

cac acc acc gct gaa tct cac aac cgt gtg atg atc gtg gag gtc atg    652
His Thr Thr Ala Glu Ser His Asn Arg Val Met Ile Val Glu Val Met
155                 160                 165                 170

ggc cgc cac gtg ggt tgg att gct ctg cac gca ggt atg gcc ggc ggt    700
Gly Arg His Val Gly Trp Ile Ala Leu His Ala Gly Met Ala Gly Gly
                175                 180                 185

gct cac tac acc gtt att cca gaa gta cct ttc gat att gca gag atc    748
Ala His Tyr Thr Val Ile Pro Glu Val Pro Phe Asp Ile Ala Glu Ile
            190                 195                 200

tgc aag gcg atg gaa cgt cgc ttc cag atg ggc gag aag tac ggc att    796
Cys Lys Ala Met Glu Arg Arg Phe Gln Met Gly Glu Lys Tyr Gly Ile
        205                 210                 215

atc gtc gtt gcg gaa ggt gcg ttg cca cgc gaa ggc acc atg gag ctt    844
Ile Val Val Ala Glu Gly Ala Leu Pro Arg Glu Gly Thr Met Glu Leu
    220                 225                 230

cgt gaa ggc cac att gac cag ttc ggt cac aag acc ttc acg gga att    892
Arg Glu Gly His Ile Asp Gln Phe Gly His Lys Thr Phe Thr Gly Ile
235                 240                 245                 250

gga cag cag atc gct gat gag atc cac gtg cgc ctc ggc cac gat gtt    940
Gly Gln Gln Ile Ala Asp Glu Ile His Val Arg Leu Gly His Asp Val
                255                 260                 265

cgt acg acc gtt ctt ggc cac att caa cgt ggt gga acc cca act gct    988
Arg Thr Thr Val Leu Gly His Ile Gln Arg Gly Gly Thr Pro Thr Ala
            270                 275                 280

ttc gac cgt gtt ctg gcc act cgt tat ggt gtt cgt gca gct cgt gcg    1036
Phe Asp Arg Val Leu Ala Thr Arg Tyr Gly Val Arg Ala Ala Arg Ala
        285                 290                 295

tgc cat gag gga agc ttt gac aag gtt gtt gct ttg aag ggt gag agc    1084
Cys His Glu Gly Ser Phe Asp Lys Val Val Ala Leu Lys Gly Glu Ser
    300                 305                 310

att gag atg atc acc ttt gaa gaa gca gtc gga acc ttg aag gaa gtt    1132
Ile Glu Met Ile Thr Phe Glu Glu Ala Val Gly Thr Leu Lys Glu Val
315                 320                 325                 330

cca ttc gaa cgc tgg gtt act gcc cag gca atg ttt gga tagtttttcg    1181
Pro Phe Glu Arg Trp Val Thr Ala Gln Ala Met Phe Gly
                335                 340

ggcttttatc aacagccaat aacagctctt tcgcccattg aggtggaggg gctgtttttt 1241

catgccgtaa ggaaagtgca agtaagtgaa atc                              1274

<210> 2
<211> 343
<212> PRT
<213> Corynebacterium glutamicum

<400> 2
Met Arg Ile Ala Thr Leu Thr Ser Gly Gly Asp Cys Pro Gly Leu Asn
  1               5                  10                  15

Ala Val Ile Arg Gly Ile Val Arg Thr Ala Ser Asn Glu Phe Gly Ser
             20                  25                  30

Thr Val Val Gly Tyr Gln Asp Gly Trp Glu Gly Leu Leu Gly Asp Arg
         35                  40                  45

Arg Val Gln Leu Tyr Asp Asp Glu Asp Ile Asp Arg Ile Leu Leu Arg
     50                  55                  60

Gly Gly Thr Ile Leu Gly Thr Gly Arg Leu His Pro Asp Lys Phe Lys
 65                  70                  75                  80

Ala Gly Ile Asp Gln Ile Lys Ala Asn Leu Glu Asp Ala Gly Ile Asp
                 85                  90                  95

Ala Leu Ile Pro Ile Gly Gly Glu Gly Thr Leu Lys Gly Ala Lys Trp
            100                 105                 110

Leu Ser Asp Asn Gly Ile Pro Val Val Gly Val Pro Lys Thr Ile Asp
        115                 120                 125

Asn Asp Val Asn Gly Thr Asp Phe Thr Phe Gly Phe Asp Thr Ala Val
    130                 135                 140

Ala Val Ala Thr Asp Ala Val Asp Arg Leu His Thr Thr Ala Glu Ser
145                 150                 155                 160

His Asn Arg Val Met Ile Val Glu Val Met Gly Arg His Val Gly Trp
                165                 170                 175

Ile Ala Leu His Ala Gly Met Ala Gly Gly Ala His Tyr Thr Val Ile
            180                 185                 190

Pro Glu Val Pro Phe Asp Ile Ala Glu Ile Cys Lys Ala Met Glu Arg
        195                 200                 205

Arg Phe Gln Met Gly Glu Lys Tyr Gly Ile Ile Val Val Ala Glu Gly
    210                 215                 220

Ala Leu Pro Arg Glu Gly Thr Met Glu Leu Arg Glu Gly His Ile Asp
225                 230                 235                 240

Gln Phe Gly His Lys Thr Phe Thr Gly Ile Gly Gln Gln Ile Ala Asp
                245                 250                 255

Glu Ile His Val Arg Leu Gly His Asp Val Arg Thr Thr Val Leu Gly
            260                 265                 270

His Ile Gln Arg Gly Gly Thr Pro Thr Ala Phe Asp Arg Val Leu Ala
        275                 280                 285

Thr Arg Tyr Gly Val Arg Ala Ala Arg Ala Cys His Glu Gly Ser Phe
    290                 295                 300

Asp Lys Val Val Ala Leu Lys Gly Glu Ser Ile Glu Met Ile Thr Phe
305                 310                 315                 320

Glu Glu Ala Val Gly Thr Leu Lys Glu Val Pro Phe Glu Arg Trp Val
                325                 330                 335

Thr Ala Gln Ala Met Phe Gly
            340